# EUROPÄISCHE PATENTANMELDUNG

(11) **EP 3 081 235 A1**
(43) Veröffentlichungstag der Anmeldung: **19.10.2016**
(21) Anmeldenummer: 15163646.1
(22) Anmeldetag: 15.04.2015
(51) Int. Cl.: A61L 9/04, A61L 9/12, B65D 85/00

(54) **RAUMDUFT-SPENDER**

(71) Anmelder: Tomil s.r.o., 566 01 Vysoké Myto (CZ)
(72) Erfinder: Müller, Tomás, 50008 Hradec Kralove (CZ)
(74) Vertreter: dompatent von Kreisler Selting Werner - Partnerschaft von Patent- und Rechtsanwälten mbB

(57) **Zusammenfassung**

Raumduft-Spender, mit einem Gehäuse (12), einem im oder am Gehäuse (12) anbringbaren Dufteinsatz (14), wobei der Dufteinsatz (14) ein formstabiles Grundelement (16), das einen Aufnahmeraum und ein in dem Aufnahmeraum befindliches Duftgel (18) aufweist, wobei das Gehäuse (12) mindestens ein Halteelement (20a, 20b, 20c, 20d) zum Halten des Grundelements (16) des Dufteinsatzes (14) aufweist, derart, dass der Dufteinsatz (14) im oder am Gehäuse (12) gehalten wird.

## Beschreibung

Die Erfindung betrifft einen Raumduft-Spender.

Aus dem Stand der Technik sind Raumduft-Spender bekannt, die über ein Befestigungselement beispielsweise an einer Raumwand befestigt werden können. Es ist bekannt Raumduft-Spender zu verwenden, die eine Duftflüssigkeit enthalten. Diese kann beispielsweise in einer auswechselbaren Flasche angeordnet sein, die von einem Gehäuse an der Raumwand gehalten wird. Durch das Verdunsten der Duftflüssigkeit wird ein Duft abgegeben. Sobald die Duftflüssigkeit vollständig verdunstet ist, kann die Flasche mit der Duftflüssigkeit gegen eine neue ausgetauscht werden, so dass der Raumduft-Spender weiter verwendet werden kann.

Nachteilig an Raumduftspendern, bei denen eine Duftflüssigkeit verwendet wird, ist die grundsätzlich schwierige Handhabung von Flüssigkeiten. Beispielsweise kann es vorkommen, dass ein Benutzer, sobald er die Duftflasche öffnet, einen Teil der Duftflüssigkeit verschüttet. Ferner nachteilig ist, dass Flüssigkeiten grundsätzlich relativ schnell verdunsten, was zu einer begrenzten Wirkungsdauer von Raumduft-Spendern mit Duftflüssigkeiten führt.

Aufgabe der Erfindung ist es, einen Raumduft-Spender bereitzustellen, der eine vereinfachte Handhabung aufweist.

Die Lösung der Aufgabe erfolgt erfindungsgemäß durch die Merkmale des Anspruchs 1.

Der erfindungsgemäße Raumduft-Spender weist ein Gehäuse und einen im oder am Gehäuse anbringbaren Dufteinsatz auf. Der Dufteinsatz weist ein formstabiles Grundelement auf, das wiederum einen Aufnahmeraum aufweist. In dem Aufnahmeraum ist ein Duftgel angeordnet. Dieses ist vorzugsweise pastös ausgebildet. Dies bedeutet, dass das Duftgel bei Raumtemperatur eine derartige Konsistenz, insbesondere Viskosität und/oder Fließfähigkeit aufweist, dass es allein aufgrund der Schwerkraft nicht aus dem Aufnahmeraum herausfließt und zwar unabhängig davon, in welcher Position das Grundelement relativ zur Schwerkraftrichtung ausgerichtet wird.

Das Gehäuse weist ferner mindestens ein Halteelement zum Halten des Grundelements des Dufteinsatzes auf. Hierdurch wird das Grundelement derart gehalten, dass der Dufteinsatz im oder am Gehäuse gehalten wird.

Durch die Verwendung eines Duftgels kann eine einfachere Handhabung des Raumduftspenders für den Benutzer erreicht werden, da bei einem Duftgel nicht die Gefahr besteht, dass dieses durch einen Benutzer verschüttet wird. Ferner kann eine längere Duftabgabe erreicht werden, da ein Duftgel nicht so schnell wie eine Flüssigkeit verdunsten wird. Durch die Verwendung eines formstabilen Grundelements zum Aufnehmen des Duftgels wird dennoch ein einfacher Austausch des Dufteinsatzes nachdem das Duftgel verbraucht ist ermöglicht. Hierzu kann der Dufteinsatz vom Benutzer am formstabilen Grundelement gehalten werden, so dass der Benutzer das Duftelement nicht berühren muss. Der verbrauchte Dufteinsatz kann somit auf einfache Weise aus dem Gehäuse herausgenommen werden und durch einen neuen ersetzt werden. Dieser wiederum wird erneut von dem mindestens einen Halteelement im Gehäuse gehalten.

Es ist bevorzugt, dass das Gehäuse mindestens zwei Halteelemente aufweist, die als Klemmelemente zum klemmenden Halten des Dufteinsatzes ausgebildet sind.

Beispielsweise kann das Grundelement als Rechteck oder Quadrat mit insbesondere abgerundeten Ecken ausgebildet sein.

In diesem Fall kann das Gehäuse vier als Klemmelemente ausgebildete Halteelemente aufweisen. Durch diese erfolgt ein klemmendes Halten der vier Ecken des Grundelements des Dufteinsatzes.

Weiterhin ist es bevorzugt, dass das Gehäuse mindestens zwei Abstandhalterelemente aufweist, an denen der Dufteinsatz anliegt, wenn er von den vier Klemmelementen gehalten wird. Alternativ kann das Grundelement mindestens zwei Abstandhalterelemente aufweisen, die am Gehäuse anliegen, wenn der Dufteinsatz von den Klemmelementen gehalten wird. Beispielsweise kann das Grundelement an jeder seiner vier Ecken einen nach außen vorstehenden Ansatz aufweisen, der an jeweils einem Klemmelement anliegt, so dass die Distanz mit der der Dufteinsatz in die vier Klemmelemente eingeschoben werden kann, genau definiert wird.

Weiterhin kann das Gehäuse eine schlitzförmige Öffnung aufweisen, durch die der Dufteinsatz in das Gehäuse hineinschiebbar und aus diesem herausziehbar ist. Hierdurch wird es möglich den Dufteinsatz beispielsweise in einer Verkaufsposition derart zu positionieren, dass er teilweise aus dem Gehäuse herausragt. In dieser Position kann der erfindungsgemäße Raumduftspender in einer Verkaufsverpackung einem Käufer präsentiert werden. Dies ist besonders ansprechend, da der Käufer den vorzugsweise mit farbigem Duftgel gefüllten Dufteinsatz zum Teil außerhalb des Gehäuses erkennen kann und mit diesem eine besondere Frische- oder Duftwirkung assoziiert. Durch das teilweise Präsentieren des Dufteinsatzes außerhalb des Gehäuses kann somit eine Verkaufsförderung erreicht werden.

Es ist bevorzugt, dass das Gehäuse ein Rückelement und ein aufklappbares Deckelelement aufweist, das über ein Gelenk, beispielsweise ein Festkörpergelenk, am Rückelement angebracht ist. Durch das aufklappbare Deckelelement wird ein einfacher Austausch des verbrauchten Dufteinsatzes gegen einen neuen ermöglicht.

In einer bevorzugten Ausführungsform weist eines der Elemente aus Vorderund Rückelement die vier Klemmelemente auf. Das jeweils andere Element aus Vorder- und Rückelement weist mindestens ein Positionierelement auf, durch das der Dufteinsatz in seiner Position zwischen den vier Klemmelementen gehalten wird. Hierdurch wird ein Herausrutschen des Dufteinsatzes aus den vier Klemmelementen verhindert. Der Dufteinsatz kann somit nur dann aus den vier Klemmelementen herausgenommen werden, wenn das Deckelelement aufgeklappt ist.

Es ist bevorzugt, dass das Positionierelement gleichzeitig als Anschlagelement dient, durch das der Dufteinsatz in seiner Position gehalten wird, in der er teilweise aus der schlitzförmigen Öffnung herausragt (Verkaufsposition). Somit kann sichergestellt werden, dass der Dufteinsatz über eine genau definierte Distanz aus dem Gehäuse herausragt und somit einem potentiellen Käufer in einer Verkaufsverpackung präsentiert werden kann. Beispielsweise kann es sinnvoll sein, den Dufteinsatz über ca. ein Viertel seiner Länge aus dem Gehäuse herausragen zu lassen. Dies ist in der Regel ausreichend, damit ein potentieller Käufer die Farbe des Duftgels gut erkennen kann, wodurch bereits ein verkaufsfördernder Effekt erreicht werden kann.

Im Folgenden werden bevorzugte Ausführungsformen der Erfindung anhand von Figuren erläutert.

Es zeigen:
- Fig. 1: eine Schrägansicht von oben einer ersten Ausführungsform des erfindungsgemäßen Duftspenders,
- Fig. 2: eine Schrägansicht derselben Ausführungsform des Duftspenders in aufgeklapptem Zustand,
- Fig. 3: eine Ansicht von unten derselben Ausführungsform des Duftspenders,
- Fig.4: eine Schrägansicht von unten derselben Ausführungsform des Duftspenders,
- Fig. 5: eine Schrägansicht des aufgeklappten Gehäuses,
- Fig. 6: eine Schrägansicht eines herausgenommenen Dufteinsatzes,
- Fig. 7: eine Ansicht von unten eines herausgenommenen Dufteinsatzes,
- Fig. 8: eine Ansicht von oben des Gehäuses mit teilweise aus diesem herausgezogenem Dufteinsatz,
- Fig. 9: eine Schrägansicht des Gehäuses mit ausgezogenem Dufteinsatz,
- Fig. 10: ein aufgeklapptes Gehäuse mit herausgezogenem Dufteinsatz.

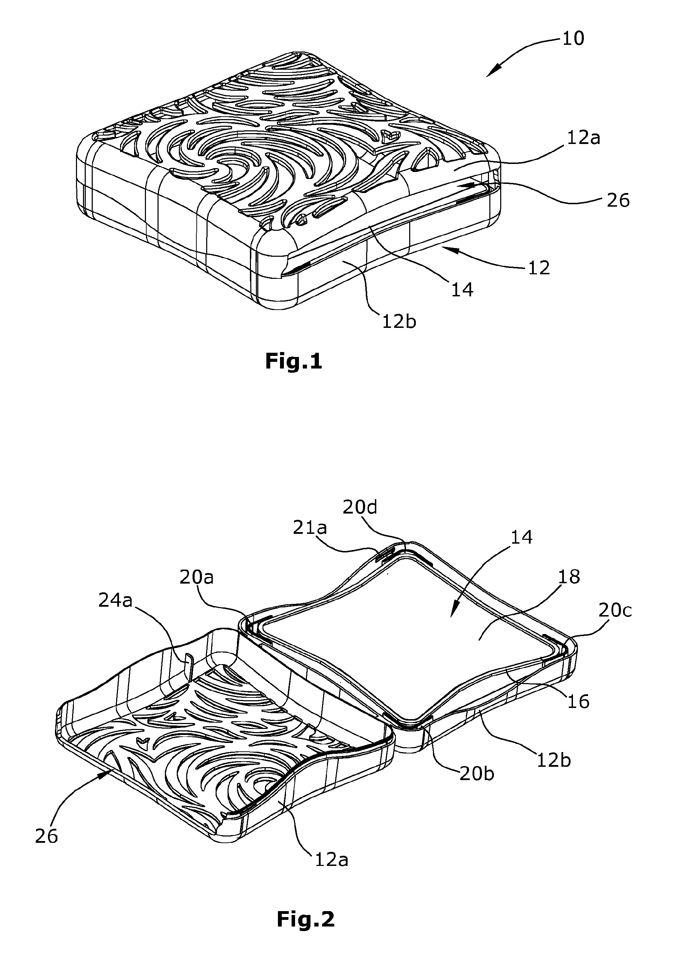

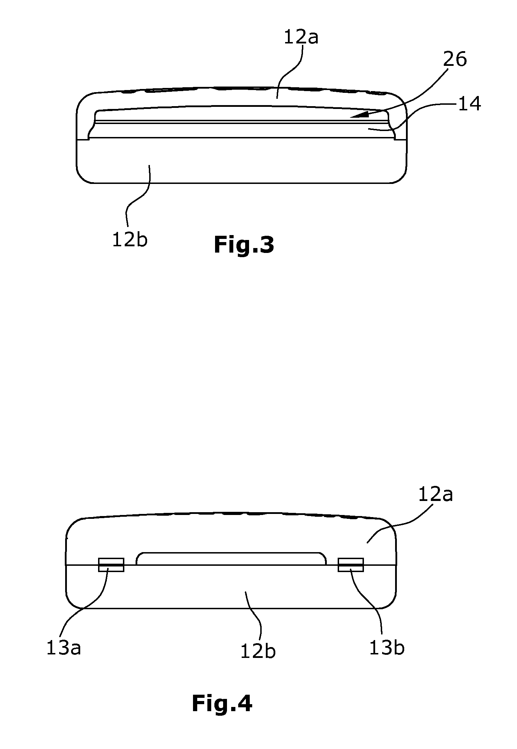

Wie in Fig. 1 sichtbar weist der erfindungsgemäße Duftspender 10 ein Gehäuse 12 mit einem in diesem anbringbaren Dufteinsatz 14 auf. Das Gehäuse 12 weist ein Rückelement 12b und ein aufklappbares Deckelelement 12a auf. Wie in Fig. 4 erkennbar kann dieses Deckelelement über zwei Festkörpergelenke 13a, 13b mit dem Rückelement 12b verbunden sein.

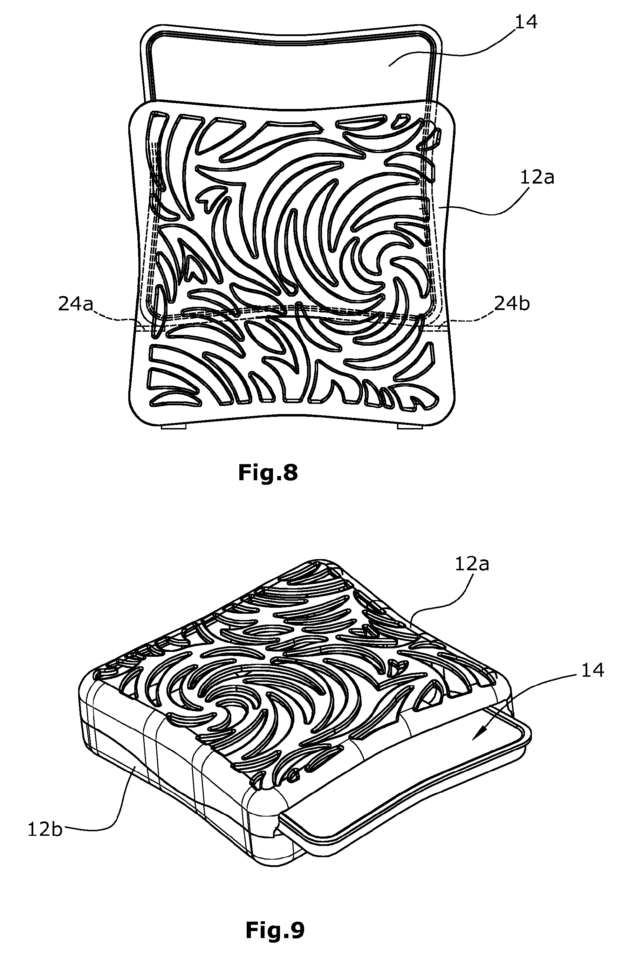

Das Gehäuse 12 weist eine schlitzförmige Öffnung 26 auf. Diese ist derart ausgebildet, dass der Dufteinsatz 14 teilweise aus dem Gehäuse 12 herausgezogen werden kann und somit durch die Öffnung 26 aus dem Gehäuse 12 herausragt. Diese Position wird als Verkaufsposition bezeichnet und ist in den Fign. 8 bis 10 dargestellt.

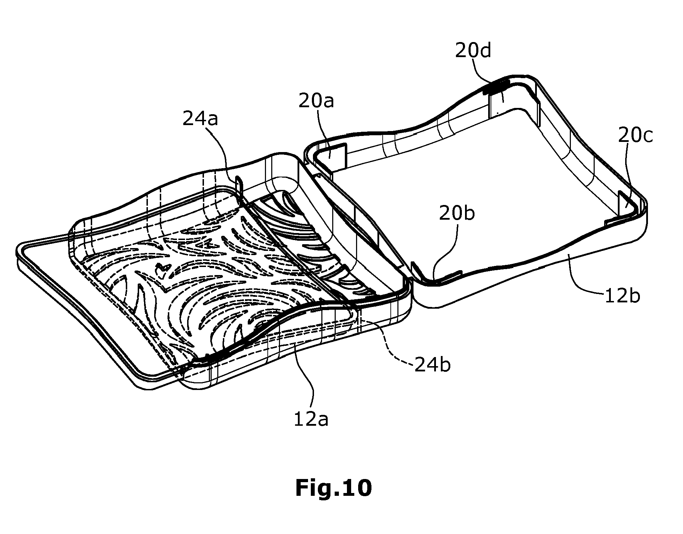

In Fig. 8 und 9 ist erkennbar, dass ein Teil des Dufteinsatzes 14 in dieser Verkaufsposition aus dem Gehäuse herausragt. Um die Distanz, über die der Dufteinsatz 14 aus dem Gehäuse 12 herausragt, genau definieren zu können, werden die beiden Positionierelemente 24a, 24b, die im Deckelelement 12a ausgebildet sind, als Anschlagelemente verwendet, durch die der Dufteinsatz 14 in einer definierten Position gehalten wird, in der er teilweise aus der schlitzförmigen Öffnung 26 herausragt (Verkaufsposition). Hierbei liegt das formstabile Grundelement 16 an den beiden Anschlagelementen 24a, 24b an, so dass verhindert wird, dass der Dufteinsatz 14 weiter in das Gehäuse 12 hineingeschoben werden kann. Ein weiteres Hineinschieben des Dufteinsatzes 14 in das Gehäuse 12 ist nur möglich, wenn der Dufteinsatz 14 bei aufgeklapptem Gehäuse 12 (siehe Fig. 10) aus dem Deckelelement 12a herausgenommen wird, über die Anschlagelemente 24a, 24b hinweggehoben wird und vollständig in das Gehäuse eingeführt wird.

Hierbei wird der Dufteinsatz vorzugsweise gewendet. Dies bedeutet, dass die Frontseite des Dufteinsatzes (das heißt die Seite des formstabilen Grundelements 16) in der Verkaufsposition in Richtung des ersten Deckelelements 12a zeigt und somit vorzugsweise in einer Verkaufsverpackung nach vorne sichtbar ist. Dies ist vorteilhaft, da in der Verkaufsposition die Rückseite des Dufteinsatzes mit einem Abdeckelement beispielsweise eine Folie abgedeckt ist, so dass das im Aufnahmeraum des Grundelements 16 befindliche Duftgel 18 geschützt ist. Zum Benutzen des Duftspenders 10 zieht der Benutzer diese nicht dargestellte Abdeckfolie ab, dreht den Dufteinsatz um 180 Grad und positioniert diesen mit seiner Vorderseite in Richtung des Rückelements 12b zwischen den vier Klemmelementen 20a bis 20d. In dieser Position (Benutzungs-Position) ragt die offene Hinterseite des Dufteinsatzes 14 (da heißt die Seite des Aufnahmeraums) in Richtung des Frontelements 12a. Diese Position ist in Fig. 2 dargestellt. Hier ist erkennbar, dass das Deckelelement 12a eine Vielzahl von Ausnehmungen aufweist, durch die der Duft des Duftgels aus dem Gehäuse 12 in den Raum entweichen kann.

In der Benutzungs-Position dienen die Positionierelemente 24a, 24b nicht mehr als Anschlagelemente (wie in der Verkaufsposition), sondern sie dienen als Positionierelemente und halten den Dufteinsatz 14 in seiner in Fig. 2 dargestellten Position, in der der er zwischen den vier Klemmelementen 20a-20d gehalten wird.

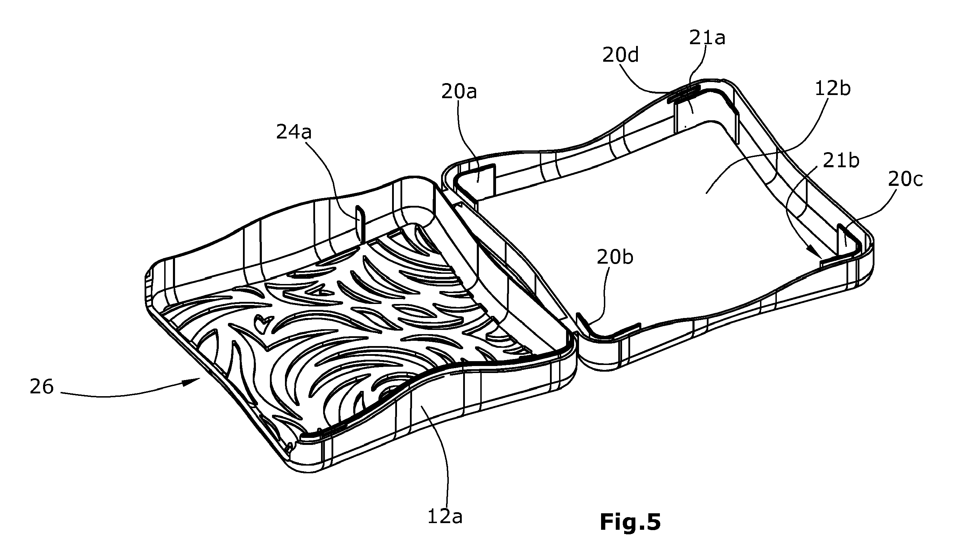

Diese vier Klemmelemente 20a bis 20d sind in Fig. 5 besser erkennbar. Hierbei handelt es sich um jeweils einen Klemmsteg, der ausgehend vom Rückelement 12b in Richtung des Deckelelements 12a verläuft und sich vorzugsweise ausgehend vom Rückelement 12d in einem rechten Winkel erstreckt. Diese vier Klemmstege 20a bis 20d sind vorzugsweise L-förmig ausgebildet, so dass sich jeder von ihnen um eine vorzugsweise abgerundete Ecke des formstabilen Grundelements 16 herumlegen kann. Dementsprechend sind die Klemmelemente 20a bis 20d vorzugsweise auch als abgerundete L-förmige Klemmelemente ausgebildet. Damit das Deckelelement 12a in geschlossenem Zustand des Gehäuses 12 am Rückelement 12b gehalten wird und nicht aufklappt, wenn das Gehäuse 12 beispielsweise an einer Raumwand befestigt ist, kann das Rückelement 12b zwei Nuten 21a, 21b aufweisen, in die korrespondierende Vorsprünge an den seitlichen Wänden des Deckelelements 12a (in Figur 5 nicht dargestellt) eingreifen. Somit wird das Deckelelement 12a formschlüssig am Rückelement 12b gehalten.

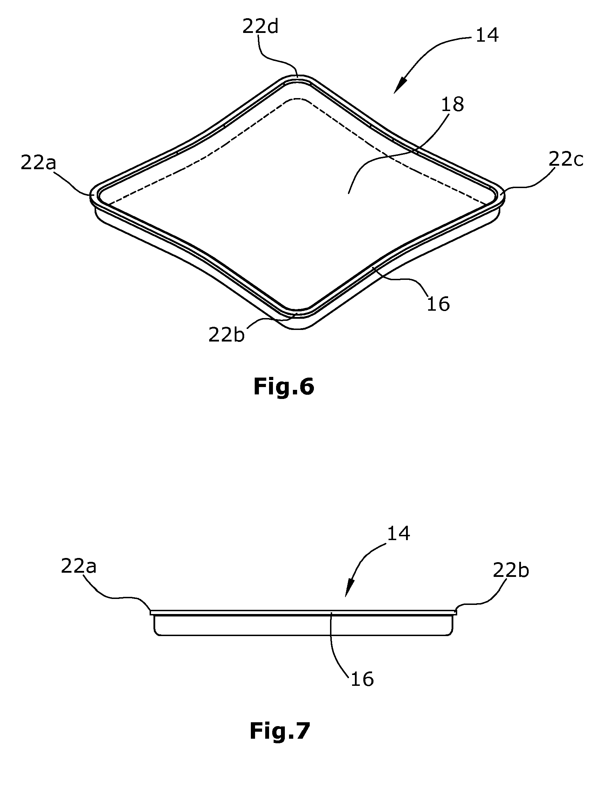

Um zu verhindern, dass der Dufteinsatz 14 zu weit in die Klemmelemente 20a bis 20d hereingedrückt wird, kann das formstabile Grundelement 16 vier Abstandhalterelemente 22a, 22b, 22c, 22d aufweisen, die vorzugsweise an jeder seiner Ecken ausgebildet sind. Dies ist in den Fign. 6 und 7 erkennbar. Vorzugsweise kann das formstabile Grundelement 16 einen umlaufenden Steg aufweisen, der vollständig um den Umfang des Grundelements 16 herum verläuft und durch den an den vier Ecken des Grundelements 16 die Abstandhalterelemente 22a bis 22d ausgebildet sind. Diese liegen in der Position gemäß Fig. 2 an den Klemmelementen 20a bis 20d an, so dass der Dufteinsatz 14 in einer genau definierten Position zwischen den Klemmelementen gehalten wird.

## Patentansprüche

1. Raumduft-Spender, mit
einem Gehäuse (12),
einem im oder am Gehäuse (12) anbringbaren Dufteinsatz (14),
wobei der Dufteinsatz (14) aufweist:
ein formstabiles Grundelement (16), das einen Aufnahmeraum aufweist,
einem in dem Aufnahmeraum befindlichen Duftgel (18),
wobei das Gehäuse (12) mindestens ein Halteelement (20a, 20b, 20c, 20d) zum Halten des Grundelements (16) des Dufteinsatzes (14) aufweist, derart, dass der Dufteinsatz (14) im oder am Gehäuse (12) gehalten wird.

2. Raumduft-Spender nach Anspruch 1, **dadurch gekennzeichnet, dass** das Gehäuse (12) mindestens zwei Halteelemente (20a-20d) aufweist, die als Klemmelemente zum klemmenden Halten des Dufteinsatzes (14) ausgebildet sind.

3. Raumduft-Spender nach Anspruch 1 oder 2, **dadurch gekennzeichnet, dass** das Grundelement (16) als Rechteck oder Quadrat mit insbesondere abgerundeten Ecken ausgebildet ist.

4. Raumduft-Spender nach Anspruch 2 und 3, **dadurch gekennzeichnet, dass** das Gehäuse (12) vier als Klemmelemente ausgebildete Halteelemente (20a-20d) aufweist zum klemmenden Halten der vier Ecken des Grundelements (16) des Dufteinsatzes (14).

5. Raumduft-Spender nach Anspruch 4, **dadurch gekennzeichnet, dass** das Gehäuse (12) mindestens zwei Abstandhalteelemente (22a, 22b) aufweist, an denen der Dufteinsatz (14) anliegt, wenn er von den vier Klemmelementen (20a-20d) gehalten wird oder das Grundelement (16) mindestens zwei Abstandhalteelemente (22a-22d) aufweist, die am Gehäuse (12) anliegen, wenn der Dufteinsatz (14) von den Klemmelementen (20a-20d) gehalten wird.

6. Raumduft-Spender nach einem der Ansprüche 1 bis 5, **dadurch gekennzeichnet, dass** das Gehäuse (12) eine schlitzförmige Öffnung (26) aufweist, durch die der Dufteinsatz (14) in das Gehäuse (12) hineinschiebbar und aus diesem herausziehbar ist.

7. Raumduft-Spender nach einem der Ansprüche 1 bis 6, **dadurch gekennzeichnet, dass** das Gehäuse (12) ein Rückelement (12a) und ein aufklappbares Deckelelement (12b) aufweist, das über ein Gelenk am Rückelement (12a) angebracht ist.

8. Raumduft-Spender nach Anspruch 4 und 7, **dadurch gekennzeichnet, dass** eines der Elemente aus Vorder- und Rückelement (12a, 12b) die vier Klemmelemente (20a-20d) aufweist und das jeweils andere Element aus Vorder- und Rückelement (12a, 12b) mindestens ein Positionierelement (24) aufweist, durch das der Dufteinsatz (14) in seiner Position zwischen den vier Klemmelementen (20a-20d) gehalten wird.

9. Raumduft-Spender nach Anspruch 6 und 8, **dadurch gekennzeichnet, dass** das Positionierelement (24) gleichzeitig als Anschlagelement dient, durch das der Dufteinsatz (14) in einer Position gehalten wird, in der er teilweise aus der schlitzförmigen Öffnung (26) herausragt.
